# EUROPÄISCHE PATENTSCHRIFT

(11) **EP 1 183 996 B1**
(45) Veröffentlichungstag und Bekanntmachung des Hinweises auf die Patenterteilung: **17.03.2004**
(21) Anmeldenummer: 00118167.6
(22) Anmeldetag: 30.08.2000
(51) Int. Cl.: A61B 5/107

(54) **Gerät zur Wachstumsmessung**
Device for measuring body growth
Dispositif de mesure de la croissance d'un individu

(43) Veröffentlichungstag der Anmeldung: 06.03.2002
(73) Patentinhaber: Popov, Wesselin, 1050 Wien (AT)
(72) Erfinder: Popov, Wesselin, 1050 Wien (AT)

(56) Entgegenhaltungen:
- DE-A- 3 835 609
- GB-A- 2 130 376
- US-A- 4 518 052

## Beschreibung

Die Erfindung betrifft ein wachstumsförnderndes Gerät. Ein solches Gerät ist zwar aus der WO 92 /12761 A1 bekannt. Allerdings weist dieses Gerät eine völlig andere Konstruktion als das erfindungsgemässe auf und arbeitet nach einem ganz anderen Prinzip ( z. B . Auflegen der bekannten ,entsprechend dimensionierten Vorrichtung auf den zu behandelnden Körperbereich ). Somit konnte diese bekannte Vorrichtung kein Vorbild für die Lösung der erfindungsspezifischen Aufgabe abgeben.

Dokument US-A-4 518 052 offenbart ein Gerät, das eine mit einer Längenmessskala versehene Stange aufweist, entlang der eine Messlatte verschieb- und feststellbar ist, an der eine Zähleinrichtung, insbesondere mit digitaler Anzeige, vorgesehen ist. Dieses Gerät weist außerdem einen Druckknopf auf, der bei Berührung durch Kopf oder Fingerspitzen des sich in gestreckter Position bzw. mit gestrecktem Oberkörper befindlichen Benützers aktivierbar ist.

Dem erfindungsgemässen Gerät liegt die Aufgabe zugrunde , den Benützer mehrmals (öfters) in Zeitabständen zu einer gestreckten Körperposition bzw. in eine Körperstellung mit gestrecktem Oberkörper zu bringen, was das Wachstum fördert. Dabei soll das Gerät in vertikaler (stehender oder sitzender Benützer) oder horizontaler Lage (liegender Benützer) verwendbar sein, unter kleinstmöglichem Kostenaufwand und möglichst raumsparend untergebracht werden können.

Zur Lösung dieser Aufgabe weist das wachstumsfördernde Gerät erfindungsgemäss eine mit einer Längenmessskala versehene Stange aufweist, entlang der eine Messlatte (Messschlitten) verschieb- und festellbar ist ,an der eine Zähleinrichtung, insbesondere mit digitaler Anzeige, vorgesehen ist, wobei die Messlatte einen Druckknopf aufweist , der bei Berührung durch Kopf, Füsse oder Fingerspitzen des sich in gestreckter Position bzw . mit gestrecktem Oberkörper befindlichen Benützers aktivierbar ist und darauf hin die Zähleinrichtung zur Anzeige der erfolgreichen Betätigungsversuche aktiviert.

Aus dem in der DE 38 35 609 A beschriebenen Längenmessgerät geht als bekannt hervor, entlang einer mit einer Messskala versehenen Stange eines Längenmessgerätes eine Messlatte (Messschlitten) verschieb- und feststellbar anzuordnen , an der (dem) eine Zähleinrichtung insbesondere mit digitaler Anzeige vorgesehen ist. Bei diesem bekannten Gerät handelt es sich wie erwähnt um ein bloßes Gerät zum Messen der Körperlänge des Benützers, bei dem sich der Benützer nicht wie beim erfindungsgemässen Gerät strecken soll. Ansonsten wäre das Messergebnis eher falsch und unerwünscht. Insbesondere ist das Gerät für Personen geeignet, die sich im Wachstum befinden (4 bis 21 -jährige). Durch die ständige Verwendung des erfindungssgemässen Gerätes werden außerdem Wirbelsäule und Halswirbel zu einer gesünderen Haltung angeregt.

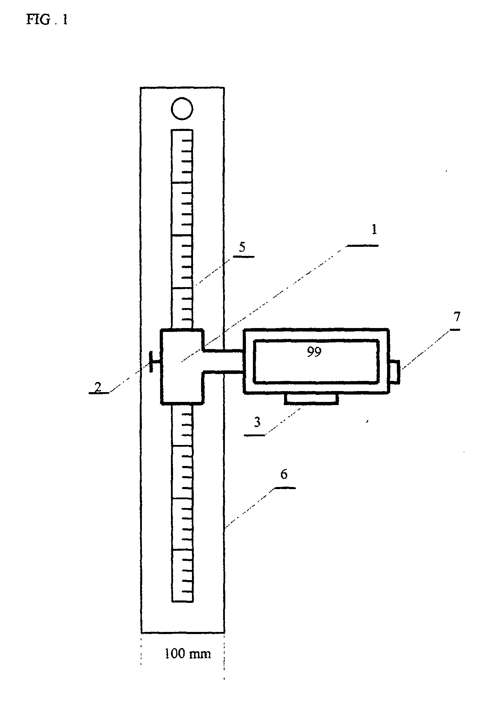

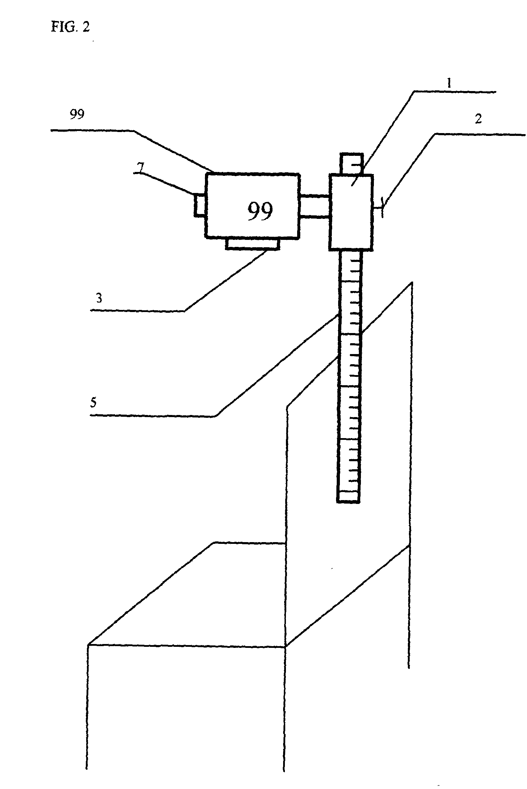

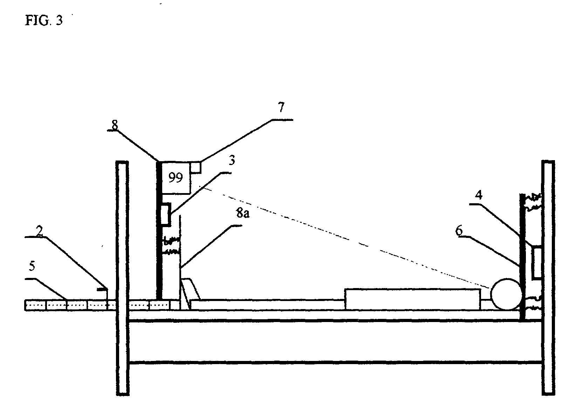

Weitere vorteilhafte Einzelheiten der Erfindung sind nachstehend anhand der Zeichnung näher erläutert. Es zeigen : Fig. 1 das an einer Befestigungslatte angeordnete wachstumsfördernde Gerät in Seitenansicht und Fig . 2 das Gerät gemäß Fig. 1 an einer Sessellehne befestigt, in Schrägansicht sowie Fig . 3 das wachstumsfördernde Gerät einen in einem Bett liegenden Benützer in Seitenansicht und eher schematisch dargestellt. Das Gerät besteht aus einer mit einer Längenmessskala 5 (in mm ) versehenen Stange, entlang der eine Messlatte (1) (Messschlitten ) verschieb- und mittels Fixierschraube (2) in einer gewünschten Position feststellbar ist. Die Längenmessskala (5) kann aus Leichtmetall oder Edelstahl in Form eines Profilstückes oder Rohres hergestellt sein. Die Stange ist an einer Grundplatte angeordnet, die beispielsweise an einer Wand für stehende Personen (Fig. 1 ) oder einer Stuhllehne (Fig. 2) für sitzende Personen in verschiedenen Höhen je nach der Körperlänge des Benützers befestigbar ist. Das wachstumsfördernde Gerät ist auch an einem Bett befestigbar ( Fig . 3) , wodurch es sowohl zu Hause als auch in Krankenhäusern verwendbar ist. Die Grundplatte kann aus Leichtmetall, Edelstahl, Kunststoff oder Holz ( Edelholz, z.B . 1300 mm lang ) gefertigt sein. Im Fall der Fig. 3 ist am Bettkopf - und fussseitig je eine vertikale Holzplatte 6, 8 angebracht , wobei der Kopf des Benützers an der federnd am Bettende befestigten Holzplatte (6) anliegt. Dahinter befinden sich ein Druckknopf 4 zum Betätigen . Der sich an der Platte 6 mit dem Kopf abstützende Benützer streckt seinen Körper und versucht den an der Platte 8 angeordneten Druckknopf (3) für die Messeinrichtung mit der vom Kopfende des Bettes aus lesbaren, erhöht an der Platte 8 angeordneten Digitalanzeige (99) auszulösen. Wie aus Fig. 3 ersichtlich ist, liegen die Füße des Benützers an einer flexiblen Platte (8a) an, die federnd an der zugehörigen Holzplatte (8) befestigt ist . Gelingt ihm dies , so wird ein akustisches Signal ausgelöst und Digitalziffern geben bekannt, daß bzw. wie der Benützer sein Ziel erreicht hat.

Die "Messlatte" ( Messschlitten ) enthält ein Display mit einer Zähleinrichtung , das die erfolgreichen Betätigungsvorgänge anzeigt, wenn der Benützer mit seinem Kopf die verschiebbare Messlatte berührt . Die Zähleinrichtung wird durch einen Druckknopf (3) infolge Berührung des Kopfes oder der Fingerspitzen des Benützers aktiviert . Mittels einem Knopf (7) kann die digitale Anzeige mit maximal 99 Auslösevorgängen wieder auf den Stand Null gebracht werden. In der Digitaleinrichtung sind auch noch andere Daten wie etwa Datum und jeweils gemessene Körperlänge speicherbar, um so besser das zunehmende Wachstum ( Körperlänge ) des Benützers längere Zeiträume hindurch beobachten zu können. Als Lohn des erreichten Zieles kann die Digitaleinrichtung eine kleine Süßigkeit (z. B . Kaugummi ) oder ein Geldstück auswerfen. Die Körperlänge des Benützers ist außerdem entweder auf der Längenmessskala oder durch digitale Zahlenangabe auf dem Display der Zähleinrichtung ablesbar. Die Messeinrichtung wird mit Batterien (3V, 6V oder mit 9 V) betrieben , wodurch unangenehme Stromschläge nicht möglich sind.

Nach mehrfachem Erreichen der eingestellten gewünschten Höhe hat der Benützer die Möglichkeit, die Messlatte (1) um einen oder mehrere mm weiter nach oben zu verschieben und sich zu bemühen, die neue Höhe mit dem Kopf zu erreichen.

## Patentansprüche

1. Wachstumsförderndes Gerät, das eine mit einer Längenmessskala (5) versehene Stange aufweist, entlang der eine Messlatte (1) verschieb- und feststellbar ist, an der eine Zähleinrichtung , insbesondere mit digitaler Anzeige (99), vorgesehen ist, wobei die Messlatte (1) einen Druckknopf (3) aufweist, der bei Berührung durch Kopf, Füsse oder Fingerspitzen des sich in gestreckter Position bzw. mit gestrecktem Oberkörper befindlichen Benützers aktivierbar ist und daraufhin die Zähleinrichtung zur Anzeige der erfolgreichen Betätigungsversuche aktiviert.

## Claims

1. Growth-stimulating device, consisting of a bar with height-measurement scale (5), along which there is a mobile locking measuring pole (1) with calculating mechanism and digital display (99). On the measuring pole (1) there is a pushbutton (3), which is activated on contact with the head, feet or fingertips of the user standing upright with stretched torso, whereupon the calculating mechanism signals the successful attempt for activation.

## Revendications

1. Appareil de stimulation de la croissance, constitué d'un levier avec une graduation pour mesurer la hauteur (5), le long de laquelle se trouve une bande de mesure (1) mobile à blocage, avec un organe de calcul, avec un écran d'affichage numérique (99), la bande de mesure (1) ayant un bouton (3) qui s'active au contact de la tête, des plantes des pieds ou du bout des doigts dans la position étirée et/ou avec la partie supérieure du corps étirée des personnes testées présentes et active l'installation de calcul qui enregistre une tentative réussie de mise en marche.
